# EUROPEAN PATENT APPLICATION

(11) **EP 1 818 076 A1**
(43) Date of publication of application: **15.08.2007**
(21) Application number: 05809593.6
(22) Date of filing: 28.11.2005
(51) Int. Cl.: A61N 1/30

(54) **IONTOPHORESIS DEVICE**

(30) Priority: 30.11.2004 JP 2004347814; 30.06.2005 US 172058
(71) Applicant: TRANSCUTANEOUS TECHNOLOGIES INC., Shibuya-ku, Tokyo (JP)
(72) Inventor: MATSUMURA, Akihiko, 1500022 (JP); MATSUMURA, Takehiko, 1500022 (JP); NAKAYAMA, Mizuo, 1500022 (JP); AKIYAMA, Hidero, 1500022 (JP)
(74) Representative: Schäfer, Matthias W.
(86) International application number: PCT/JP2005/021766
(87) International publication number: WO 2006/059557

(57) **Abstract**

An iontophoresis device is provided capable of suppressing the change in a composition of a drug solution and/or an electrolyte solution of a working electrode structure, thereby allowing the working electrode structure to be retained in a stable state for a long period of time. The iontophoresis device includes: an electrolyte solution holding part for holding an electrolyte solution in which an electrolyte to be dissociated, in a solution, to first electrolytic ions of a first conductivity and second electrolytic ions of a second conductivity is dissolved; and a drug solution holding part for holding a drug solution in which a drug to be dissociated, in a solution, to the drug ions of the first conductivity and drug counter ions of the second conductivity is dissolved. A first ion-exchange membrane for selectively passing the ions of the second conductivity and a porous separation membrane for blocking passage of molecules and ions each having a predetermined molecular weight or more are placed between the electrolyte solution holding part and the drug solution holding part.

## Description

### BACKGROUND OF THE INVENTION

### 1. Field of the Invention

The present invention relates to an iontophoresis device including a drug solution holding part for holding a drug solution containing a drug in a working electrode structure, and an electrolyte solution holding part for holding an electrolyte solution, the iontophoresis device being capable of suppressing the change in a composition of the drug solution in the working electrode structure and/or the electrolyte solution.

### 2. Related Background Art

JP 3030517 B, JP 2000-229128 A, JP 2000-229129 A, JP 2000-237326 A, JP 2000-237327 A, JP 2000-237328 A, JP 2000-237329 A, JP 2000-288097 A, JP 2000-288098 A, and the pamphlet of WO 03/037425, the disclosures of which are incorporated herein by reference, disclose an iontophoresis device for administering an ion-dissociable drug whose drug component is dissociated to ions (drug ions) of a positive or negative conductivity (first conductivity).

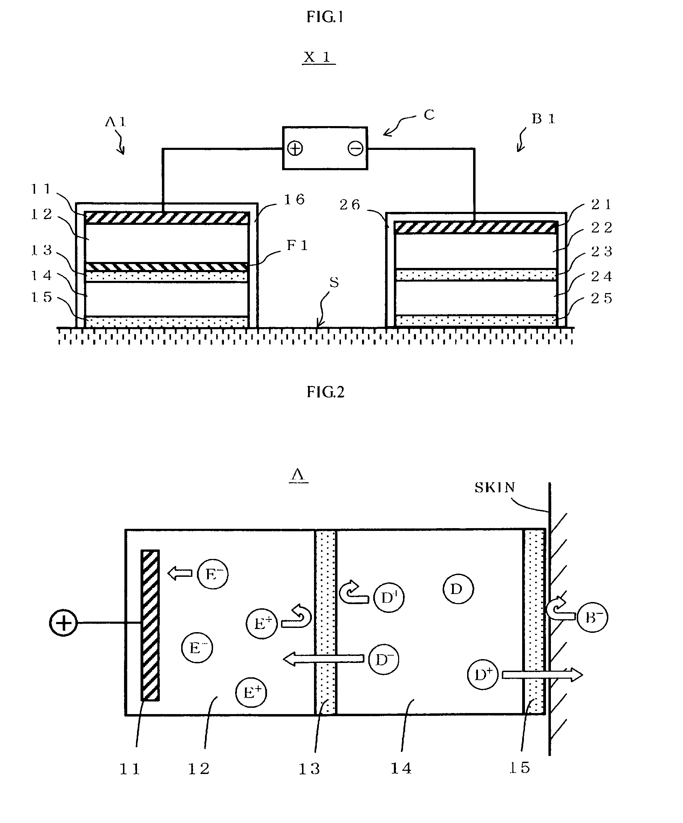

FIG. 1 is an explanatory view schematically showing the configuration and function of a working electrode structure A of the iontophoresis device.

As shown in the figure, the working electrode structure A includes:
(1) an electrode 11;
(2) an electrolyte solution holding part 12 for holding an electrolyte solution in contact with the electrode 11, in which an electrolyte dissociated to first electrolytic ions (E⁺) of a first conductivity and second electrolytic ions (E⁻) of a second conductivity opposite to the first conductivity in a solution is dissolved;
(3) a first ion-exchange membrane 13 for selectively passing the ions of the second conductivity, the first ion-exchange membranes 13 being placed on a front side of the electrolyte solution holding part 12;
(4) a drug solution holding part 14 for holding a drug solution in which a drug dissociated to drug ions (D⁺) of the first conductivity and drug counter ions (D⁻) of the second conductivity in a solution is dissolved, the drug solution holding part 14 being placed on a front side of the first ion-exchange membrane 13; and
(5) a second ion-exchange membrane 15 for selectively passing the ions of the first conductivity, the second ion-exchange membrane 15 being placed on a front side of the drug solution holding part 14.

In the iontophoresis device having the working electrode structure A, a voltage of the first conductivity (positive in the example shown in the figure) is applied to the electrode 11, whereby the drug ions (D⁺) are administered to a living body (human being or animal) via the second ion-exchange membrane 15, whereas the transfer of biological counter ions (B⁻/ions charged in a conductivity opposite to that of the drug ions and present on the surface of a living body or in the living body) to the drug solution holding part 14 is inhibited. Therefore, the drug ions can be administered to the living body efficiently. Furthermore, the transfer of the drug ions (D⁺) to the electrolyte solution holding part 12 and the transfer of H⁺ ions generated in the vicinity of the electrode 11 to the drug solution holding part 14 and then to the interface of the living body are inhibited by the first ion-exchange membrane 13. Therefore, the generation of a toxic substance owing to the electrolysis of a drug and the rapid variation in pH at the interface of the skin can be prevented.

However, in the iontophoresis device above, the following was made apparent. Depending upon the kind of an electrolyte to be used, the kind of a drug, a combination thereof, or the like, the drug may be altered with the elapse of some period of time after the assembly of the working electrode structure. Alternatively, when a drug is administered with the elapse of some period of time after the assembly of the working electrode structure, the administration efficiency of a drug may decrease remarkably compared with the administration efficiency immediately after the assembly of the working electrode structure, or a drug may be decomposed in the electrolyte solution holding part.

### SUMMARY OF THE INVENTION

The present invention has been achieved in view of the above-mentioned problems, and an object of the present invention is to provide an iontophoresis device capable of preventing or suppressing the change in a composition of a drug solution or an electrolyte solution in the case where a working electrode structure and an entire iontophoresis device including the working electrode structure are retained in an assembled state.

Another object of the present invention is to provide an iontophoresis device capable of preventing or suppressing the discoloration of a drug solution, the precipitation of crystal inadrugsolutionholdingpart, the decrease in a medical efficiency, the generation of a toxic substance due to the alteration in a drug or the like in the case where a working electrode structure and an entire iontophoresis device including the working electrode structure are retained in an assembled state.

Still another object of the present invention is to provide an iontophoresis device capable of preventing or suppressing the decrease in the administration efficiency of a drug occurring in the case where the drug is administered after a previously assembled active electrode structure or iontophoresis device is retained for a predetermined period of time or longer.

Still another object of the present invention is to provide an iontophoresis device capable of preventing or suppressing the decomposition of a drug occurring in an electrolyte solution holding part or the generation of a toxic substance caused by the decomposition in the case where the drug is administered after a previously assembled active electrode structure or iontophoresis device is retained for a predetermined period of time or longer.

Still another object of the present invention is to provide an iontophoresis device capable of storing a previously assembled active electrode structure or an entire iontophoresis device including the working electrode structure for a long period of time, thereby enabling the distribution and storage in the assembled form.

The present invention has been made to achieve the above object and, according to one aspect of the present invention, there is provided an iontophoresis device for administering drug ions to a living body, including: an electrolyte solution holding part for holding an electrolyte solution in which an electrolyte to be dissociated, in a solution, to first electrolytic ions of a first conductivity and second electrolytic ions of a second conductivity opposite to the first conductivity is dissolved; and a drug solution holding part for holding a drug solution in which a drug to be dissociated, in a solution, to the drug ions of the first conductivity and drug counter ions of the second conductivity is dissolved, the drug solution holding part being placed on a front side of the electrolyte solution holding part, in which a first ion-exchange membrane for selectively passing the ions of the second conductivity and a porous separationmembrane for blocking the passage of molecules and ions each having a predetermined molecular weight or more are placed between the electrolyte solution holding part and the drug solution holding part.

The present invention postulates an iontophoresis device including a working electrode structure similar to that of FIG. 1, that is, a working electrode structure including: (1) anelectrolyte solution holding part for holding an electrolyte solution in which an electrolyte to be dissociated, in a solution, to first electrolytic ions of a first conductivity and second electrolytic ions of a second conductivity opposite to the first conductivity is dissolved; (2) a first ion-exchange membrane for selectively passing the ions of the second conductivity, the first ion-exchange membrane being placed on a front side of the electrolyte solution holding part; and (3) a drug solution holding part for holding a drug solution in which a drug to be dissociated, in a solution, to drug ions of the first conductivity and drug counter ions of the second conductivity is dissolved, the drug solution holding part being placed on a front side of the first ion-exchange membrane .

The inventors of the present invention have found the following. As described above, when the working electrode structure with the above-mentioned configuration is left for a predetermined period of time or longer, even in the case where a stable drug that is not altered for a long period of time is used, phenomena such as the discoloration of a drug solution, the precipitation of crystal in the drug solution holding part, or the decrease in a medical efficiency/the generation of a toxic substance owing to the alteration of a drug may occur, depending upon the kind of an electrolyte, the kind of a drug, a combination thereof or the like.

The inventors of the present invention have earnestly studied based on the assumption that the above-mentioned phenomena are caused by the second electrolytic ions transferred from the electrolyte solution holding part 12 to the drug solution holding part 14. That is, the drug solution is discolored and crystal is precipitated in the drug solution holding part by the change in pH of the drug solution due to the presence of the second electrolytic ions, and the medical efficiency is decreased and the toxic substance is generated by the reaction between the second electrolytic ions and the drug.

Consequently, the inventors have found that, by blocking the transfer of the second electrolytic ions with a porous separation membrane placed between the electrolyte solution holding part and the drug solution holding part,the above-mentioned respective phenomena can be suppressed effectively, that is, the period in which the working electrode structure can be kept without each of the above-mentioned phenomena can be prolonged, thereby achieving the present invention.

The porous separation membrane (which may be called an ultrafilter, a microfilter, etc. ) in the present invention blocks the passage of molecules and ions each having a predetermined molecular weight or larger with a number of pores formed in a thin membrane. A porous separation membrane made of an arbitrary material such as, a porous membrane made of a polymer material such as polysulfone, polyacrylonitrile, cellulose acetate, polyamide, polycarbonate, or polyvinylalcohol, or a porous membrane made of a ceramics material (e.g., alumina) can be used. A porous separation membrane can be used, which has pores of an appropriate size capable of effectively blocking the transfer of the second electrolytic ions to the drug solution holding part, and allowing the transfer of drug counter ions, required for the passage of a current for the administration of a drug, to the electrolyte solution holding part.

A molecular weight cutoff is an index for representing the molecular weight of each of molecules and ions that cannot pass through the porous separation membrane. A porous separation membrane having a molecular weight cutoff larger than the molecular weight of each of drug counter ions and smaller than the molecular weight of each of the second electrolytic ions can be used as a porous separation membrane in the present invention.

The molecular weight cutoff is obtained as the molecular weight at which the blocking ratio is 90% in a cutoff curve obtained by plotting blocking ratios R (the blocking ratio R is defined by 1 - Cp/Cb, where Cb is the concentration of an electrolyte on a supply solution side via a membrane, and Cp is the concentration of an electrolyte on a permeation solution side) with respect to a plurality of marker molecules having different molecular weights. In the case where the molecular weight cutoff of the porous separation membrane used in the present invention is close to the molecular weight of the second electrolytic ions or the molecular weight of the drug counter ions, the prolongable degree of the period for keeping the working electrode structure without decreasing the conductivity in the administration of a drug, and without the discoloration, alteration, and the like of a drug may become shorter.

Further, the passage characteristics of molecules and ions with respect to the porous separation membrane are influenced by the three-dimensional shapes of molecules and ions or the like. Therefore, it is true that the molecular weight cutoff is an important index for selecting a porous separation membrane used in the present invention, but even in the case where a porous separation membrane having a molecular cutoff sufficiently larger than the molecular weight of each of drug counter ions and sufficiently smaller than the molecular weight of each of the second electrolytic ions is selected, the prolongable degree of the period for keeping the working electrode structure without the decrease of the conductivity in the administration of a drug, the discoloration, alteration, and the like of a drug may be small.

Thus, it is preferable to select the porous separation membrane used in the present invention by prototyping a working electrode structure using a porous separation membrane having a molecular weight cutoff in a range from the molecular weight of each of drug counter ions to that of each of second electrolytic ions or a molecular weight cutoff close to that range, and experimentally confirming the prolongable degree of the storage period and the current passage characteristics (conduction characteristics).

The drug ions in the present invention refer to those which are generated by dissolving a drug and bear the medical effect when administered to a living body. The drug counter ions refer to those which are charged in a conductivity opposite to that of the drug ions generated by dissolving a drug. Furthermore, in the present invention the first electrolytic ions and the second electrolytic ions respectively refer to ions charged in the same conductivity as that of the drug ions and ions charged in a conductivity opposite to that of the drug ions, each being generated by dissolving an electrolyte in the electrolyte solution holding part.

The phrase "blocking the passage of molecules or ions" in the present specification does not necessarily mean the complete blocking. The phrase includes the case where the transfer of the second electrolytic ions is limited to such a degree that the working electrode structure can be kept without causing the phenomena such as the discoloration and alternation of a drug for a period of time required in terms of use, for example, even when the second electrolytic ions are transferred to the drug solution holding part at a certain speed. Similarly, the phrase "allowing the passage of molecules or ions" does not mean that the passage of molecules and ions is not limited at all. The phrase includes the case where the passage of drug counter ions is kept to such a degree that the passage of a current is exhibited so as not to impair the use, even when the transfer speed of the drug counter ions to the electrolyte solution holding part decrease to some degree.

An electrolyte solution in which two or more kinds of electrolytes are dissolved may be used as the electrolyte solution in the electrolyte solution holding part for the purpose of suppressing the change in pH owing to the buffer effect and other purposes. Thus, two or more kinds of the second electrolytic ions may be present in the electrolyte solution holding part. In such a case, a porous separation membrane capable of blocking the transfer of only the second electrolytic ions causing each of the above phenomena by transferring to the drug solution holding part only needs to be used as the porous separation membrane of the present invention.

The inventors of the present invention have also found the following. Independently from the phenomena such as the discoloration of a drug solution, the precipitation of crystal in the drug solution holding part and the decrease in a medical efficiency/the generation of a toxic substance caused by the alteration of a drug, when a drug is administered after the working electrode structure shown in FIG. 1 is stored for a predetermined period of time or longer, there may be the case where the administration efficiency of a drug decreases or the drug is decomposed in the electrolyte solution holding part, depending upon the kind of an electrolyte, the kind of a drug, or a combination thereof. However, according to the present invention, those phenomena also can be suppressed by placing a porous separation membrane for blocking the passage of electrolyte molecules or drug molecules between the electrolyte solution holding part and the drug solution holding part.

Although the mechanism for the occurrence of these phenomena and the mechanism for the suppression of those phenomena by the present invention are not necessarily apparent, the following fact has been confirmed by the investigation of the inventors of the present invention. In the case where those phenomena occur in the working electrode structure with the configuration shown in FIG. 1, the first electrolytic ions or drug ions, which are supposed to be blocked by the first ion-exchange membrane, are transferred over time to the drug solution holding part or the electrolyte solution holding part, respectively.

Thus, it is considered that the electrolyte molecules present in the electrolyte solution holding part under the condition of not being dissociated or the drug molecules present in the drug solution holding part under the condition of not being dissociated are respectively transferred to the drug solution holding part or the electrolyte solution holding part to be dissociated without being restricted by the first ion-exchange membrane, which causes the above-mentioned phenomena, and these phenomena can be suppressed by blocking the transfer of the not dissociated molecules with a porous separation membrane.

A porous separation membrane made of any one of various kinds of materials can be used as the porous separation membrane herein, without any limit in the same way as the above. Generally, a porous separation membrane whose molecular weight cutoff is larger than that of drug counter ions and smaller than that of electrolyte molecules or drug molecules can be used. In practical use, it is preferable to experimentally select a porous separation membrane capable of sufficiently suppressing the decrease in an administration efficiency of a drug and the decomposition of a drug (or capable of suppressing the increase in the concentration of the first electrolytic ions in the drug solution holding part or the concentration of the drug ions in the electrolyte solution holding part to a predetermined level or less) over a required storage period of time without impairing the current passage characteristics,from porousseparation membranesselected based on the above-mentioned aspect.

Furthermore, the following case also can be considered: the first electrolytic ions or the drug ions pass through the first ion-exchange membrane to be respectively transferred to the drug solution holding part or the electrolyte solution holding part, depending upon the performance of the first ion-exchange membrane to be used. In such a case, the use of a porous separation membrane for blocking the passage of the first electrolytic ions or the drug ions as the porous separation membrane in the present invention suppresses the decrease in an administration efficiency of a drug or the decomposition of a drug.

In the case where it is necessary to suppress the decrease in an administration efficiency of a drug by using a porous separation membrane for blocking the transfer of the electrolyte molecules or the first electrolytic ions in an iontophoresis device that uses an electrolyte solution, in which two or more kinds of electrolytes are dissolved, in an electrolyte solution holding part of a working electrode structure, it is preferable to use a porous separation membrane capable of blocking the transfer of all the electrolyte molecules or first electrolytic ions.

As is apparent from the above, there may be the case where only a decrease in an administration efficiency of a drug caused by the transfer of the first electrolytic ions to the drug solution holding part during a storage period become a problem, depending upon the kind of an electrolyte or a drug, or a combination thereof (for example, the second electrolytic ions are not transferred to the drug solution holding part and the drug molecules or the drug ions are not transferred to the electrolyte solution holding part, or even when these transfers occur, phenomena that impair the use such as the discoloration, alteration, and decomposition of a drug do not occur). In such a case, a porous separation membrane capable of blocking only the passage of the electrolyte molecules (or the first electrolytic ions) only needs to be selected.

Similarly, in the case where only the decomposition of a drug in the electrolyte solution holding part caused by the transfer of the drug ions to the electrolyte solution holding part during a storage period becomes a problem, a porous separation membrane capable of blocking only the passage of the drug molecules (or drug ions) only needs to be selected. In the case where only the discoloration, alteration, and the like of a drug caused by the transfer of the second electrolytic ions to the drug solution holding part become a problem, a porous separation membrane capable of blocking only the passage of the second electrolytic ions only needs to be selected.

Furthermore, in the case where the phenomena that impair the use among the above-mentioned phenomena occur in a combined manner, it is preferable that a porous separation membrane capable of blocking the passage of all the molecules or ions causing those phenomena be selected.

The present invention also can be configured in such a manner that a porous separation membrane formed in a bag shape is used, and the electrolyte solution holding part or the drug solution holding part is sealed with the bag-shaped porous separation membrane. By this feature, the following additional effects can be obtained. The convenience of the storage and transportation of the electrolyte solution holding part or the drug solution holding part, and the workability during the assembly of the working electrode structure are enhanced, and furthermore, the mixing of the electrolyte solution and the drug solution, which may occur at an end face of the first ion-exchange membrane or the porous separation membrane, can be prevented.

According to another aspect of the present invention, there is provided an iontophoresis device for administering drug ions to a living body, including: an electrolyte solution holding part for holding an electrolyte solution in which an electrolyte to be dissociated, in a solution, to first electrolytic ions of a first conductivity and second electrolytic ions of a second conductivity opposite to the first conductivity is dissolved; a drug solution holding part for holding a drug solution in which a drug to be dissociated, in a solution, to drug ions of the first conductivity and drug counter ions of the second conductivity is dissolved, the drug solution holding part being placed on a front side of the electrolyte solution holding part; and a first ion-exchange membrane for selectively passing ions of the second conductivity, the first ion-exchange membrane being placed between the electrolyte solution holding part and the drug solution holding part, in which the first ion-exchange membrane blocks passage of the drug molecules or the drug ions. In this case as well, the effect of the present invention similar to those described above can be achieved.

More specifically, a porous film in which pores are filled with ion-exchange resin having a function of exchanging ions of a second conductivity can be used as the first ion-exchange membrane. By using a first ion-exchange membrane with the size of each of the pores and the ion-exchange resin, or the filling ratio thereof selected appropriately, the transfer of the second electrolytic ions to the drug solution holding part can be blocked. This can prolong the period during which the working electrode structure can be retained without the discoloration of a drug solution and the precipitation of crystal in the drug solution holding part, or the decrease in a medical efficiency and the generation of a toxic substance caused by the alteration of a drug.

Similarly, by using a first ion-exchange membrane with the size of each of the pores of the porous film and the ion-exchange resin, or the filling ratio thereof selected appropriately, the transfer of electrolyte molecules or first electrolytic ions to the drug solution holding part can be blocked. In this case, it is possible to suppress the decrease in an administration efficiency of a drug caused by the competition of the first electrolytic ions with the drug ions. In the case where the trans fer of the drug molecules or the drug ions to the electrolyte solution holding part is blocked with the first ion-exchange membrane, the decomposition of the drug ions in the electrolyte solution holding part can be prevented.

In the above-mentioned respective inventions, it is possible to administer a drug under the condition that the drug solution holding part (for example, a thin film carrier such as gauze impregnated with a drug solution can be used as a drug solution holdingpart) is kept indirect contact with a living body. However, it is preferable that a second ion-exchange membrane selectively passing ions of the first conductivity be placed on a front side of the drug solution holding part, and a drug be administered via the second ion-exchange membrane. This can block the transfer of biological counter ions to the drug solution holding part, and further enhance the administration efficiency of a drug.

### BRIEF DESCRIPTION OF THE DRAWINGS

In the according drawings:
FIG. 1 is an explanatory view schematically illustrating the configuration and function of a working electrode structure of an iontophoresis device;
FIG. 2 is an explanatory view showing a basic configuration of an iontophoresis device according to an embodiment of the present invention; and
FIGS. 3A to 3E are explanatory views each showing a configuration of a working electrode structure of an iontophoresis device according to other embodiments of the present invention.

### DETAILED DESCRIPTION OF THE PREFERRED EMBODIMENTS

Hereinafter, an embodiment of the present invention will be described with reference to the drawings.

FIG. 2 is a schematic cross-sectional view showing a basic configuration of an iontophoresis device according to the present invention.

In the following description, for convenience, an iontophoresis device for administering a drug whose drug component is dissociated to plus drug ions (for example, lidocaine hydrochloride that is an anesthetic agent, carnitine chloride that is a gastrointestinal disease therapeutic agent, pancuronium bromide that is a skeletal muscle relaxant, or morphine hydrochloride that is an anesthetic agent) will be exemplified. In the case of an iontophoresis device for administering a drug whose drug component is dissociated tominus drug ions (forexample, ascorbic acid that is a vitamin agent, or lipid A used as an adjuvant for vaccine), the polarity (plus and minus) of a power source, each electrode, or each ion-exchange membrane in the following description is reversed.

As shown, an iontophoresis device X1 of the present invention includes a working electrode structure A1, a nonworking electrode structure B1, and a power source C, as main components (members). Reference numeral S denotes the skin (or a mucous membrane).

The working electrode structure A1 includes an electrode 11 connected to a positive pole of the power source C, an electrolyte solution holding part 12 for holding an electrolyte solution in contact with the electrode 11, a porous separation membrane F1 placed on a front side of the electrolyte solution holding part 12, an anion exchange membrane 13 placed on a front side of the porous separation membrane F1, a drug solution holding part 14 placed on a front side of the anion exchange membrane 13, and a cation exchange membrane 15 placed on a front side of the drug solution holding part 14. The entire active electrode structure A1 is housed in a cover or a container 16 composed of a material such as a resin film or a plastic.

On the other hand, the nonworking electrode structure B1 includes an electrode 21 connected to a negative pole of the power source C, an electrolyte solution holding part 22 for holding an electrolyte solution in contact with the electrode 21, a cation exchange membrane 23 placed on a front side of the electrolyte solution holding part 22, an electrolyte solution holding part 24 placed on a front side of the cation exchange membrane 23, and an anion exchange membrane 25 placed on a front side of the electrolyte solution holding part 24. The entire nonworking electrode structure B1 is housed in a cover or a container 26 composed of a material such as a resin film or a plastic.

In the iontophoresis device X1, those which are made of any conductive material can be used as the electrodes 11 and 21 withoutanyparticularlimit. Inparticular,aninactiveelectrode composed of carbon, platinum, or the like is used preferably, and a carbon electrode without any possibility of the elution of metal ions and the transfer thereof to a living body can be used particularly preferably.

However, an active electrode such as a silver/silver chloride couple electrode in which the electrode 11 is made of silver and the electrode 21 is made of silver chloride can also be adopted.

For example, in the case of using the silver/silver chloride couple electrode, in the electrode 11 that is a positive pole, a silver electrode and chlorine ions (Cl⁻) easily react with each other to generate insoluble AgCl as represented by Ag⁺Cl⁻→AgCl+e⁻, and in the electrode 21 that is a negative pole, chlorine ions (Cl⁻) are eluted from a silver chloride electrode. Consequently, the following effects can be obtained: the electrolysis of water is suppressed, and the rapid acidification based on H⁺ ions at the positive pole, and the rapid basification based on OH⁻ ions at the negative pole can be prevented.

In contrast, in the working electrode structure A1 and the nonworking electrode structure B1 in the iontophoresis device X1 in FIG. 2, owing to the function of the anion exchange membranes 13 and 25, and/or the cation exchange membranes 15 and 23, the rapid acidification based on H⁺ ions in the electrolyte solution holding part 12 and the rapid basification based on OH⁻ ions in the electrolyte solution holding part 22 are suppressed. Therefore, an inexpensive carbon electrode without any possibility of the elution of metal ions can be used preferably in place of the active electrode such as a silver/silver chloride couple electrode.

The electrolyte solution holding parts 12, 22, and 24 in the iontophoresis device X1 in FIG. 2 each hold an electrolyte solution so as to keep the conductivity. Phosphatebufferedsaline, physiological saline, etc. can be used as the electrolyte solution typically.

In order to more effectively prevent the generation of gas caused by the electrolytic reaction of water and the increase in a conductive resistance caused by the generation of gas, or the change in pH caused by the electrolytic reaction of water, an electrolyte that is more easily to be oxidized or reduced than the electrolytic reaction of water (oxidation at the positive pole and the reduction at the negative pole) can be added to the electrolyte solution holding parts 12 and 22. In terms of the biological safety and economic efficiency (low cost and easy a vailability) , for example, an inorganic compound such as ferrous sulfate or ferric sulfate, a medical agent such as ascorbic acid (vitamin C) or sodium ascorbate, and an organic acid such as lactic acid, oxalic acid, malic acid, succinic acid, or fumaric acid and/or a salt thereof can be used preferably. Alternatively, a combination of those substances (for example, 1: 1 mixed aqueous solution containing 1 mol (M) of lactic acid and 1 mol (M) of sodium fumarate) can also be used.

Each of the electrolyte solution holding parts 12, 22, and 24 may hold the above-mentioned electrolyte solution in a liquid state. However, the electrolyte solution holding parts 12, 22, and 24 may be configured by impregnating a water-absorbing thin film carrier made of a polymer material or the like with the above-mentioned electrolyte solution, thereby enhancing, for example, the ease of handling thereof. The same thin film carrier as that can be used in the drug solution holding part 14 can be used as the thin film carrier used herein. Therefore, the detail thereof will be described in the following description regarding the drug solution holding part 14.

The drug solution holding part 14 in the iontophoresis device X1 according to this embodiment holds at least an aqueous solution of a drug whose drug component is dissociated to plus drug ions by the dissolution, as a drug solution.

Herein, the drug solution holding part 14 may hold a drug solution in a liquid state. However, it is also possible to impregnate such a water-absorbing thin film carrier as described below with a drug solution so as to enhance the ease of handling thereof and the like.

Examples of a material that can be used for the water-absorbing thin film carrier in this case include a hydrogel body of acrylic resin (acrylhydrogelfilm), segmented polyurethane gel film, and an ion conductive porous sheet for forming a gel solid electrolyte. By impregnating the above aqueous solution at an impregnation ratio of 20 to 60%, a high transport number (high drug delivery property), e.g., 70 to 80% can be obtained.

The impregnation ratio in the present specification is represented by % by weight (i.e., 100 x (W-D)/D[%] where D is a weight in a dry state and W is a weight after impregnation). The impregnation ratio should be measured immediately after the impregnation with an aqueous solution to eliminate a chronological influence.

Furthermore, the transport number in the present specification refers to the ratio of a current that contributes to the transfer of particular ions among the whole current flowing through an electrolyte solution. The administration efficiency of a drug refers to the transport number regarding drug ions, that is, the ratio of a current that contributes to the transfer of drug ions among the whole current supplied to the working electrode structure.

Herein,theabove-mentionedacrylhydrogelfilm(forexample, available from Sun Contact Lens Co., Ltd.) is a gel body having a three-dimensional network structure (cross-linking structure). When an electrolyte solution that is a dispersion medium is added to the acrylhydrogel film, the acrylhydrogel filmbecomes a polymer adsorbenthavingion conductivity. Furthermore, the relationship between the impregnation ratio of the acrylhydrogel film and the transport number can be adjusted depending upon the size of the three-dimensional network structure and the kind and ratio of a monomer constituting a resin. The acrylhydrogel film with an impregnation ratio of 30 to 40% and a transport number of 70 to 80% can be prepared from 2-hydroxyethylmethacrylate and ethyleneglycol dimethacrylate (monomer ratio 98 to 99.5 : 0.5 to 2), and it is confirmed that the impregnation ratio and transport number are almost the same in a range of an ordinary thickness of 0.1 to 1 mm.

Furthermore, the segmented polyurethane gel film has, as segments, polyethyleneglycol (PEG) and polypropyleneglycol (PPG), and can be adjusted based on a monomer and diisocyanate constituting these segments. The segmented polyurethane gel film has a three-dimensional structure cross-linked by a urethane bond, and the impregnation ratio, transport number, and adhesion strength of the gel film can be easily adjusted by controlling the size of a network, and the kind and ratio of a monomer in the same way as in the acrylhydrogel film. When water that is a dispersion medium and an electrolyte (alkaline metal salt, etc.) are added to the segmented polyurethane gel film (porous gel film), oxygen in an ether connecting part of polyether forming a segment and an alkaline metal salt form a complex, and ions of the metal salt move to oxygen in a subsequent blank ether connecting part when a current flows, whereby the conductivity is expressed.

As the ion conductive porous sheet for forming a gel solid electrolyte, for example, there is the one disclosed in JP 11-273452 A. This porous sheet is based on an acrylonitrile copolymer, and a porous polymer with a porosity of 20 to 80%. More specifically, this porous sheet is based on an acrylonitrile copolymer with a porosity of 20 to 80% containing 50 mol% or more (preferably 70 to 98 mol%) of acrylonitrile. The acrylonitrile gel solid electrolytic sheet (solid-state battery) is prepared by impregnating an acrylonitrile copolymer sheet soluble in a non-aqueous solvent and having a porosity of 20 to 80%, with a non-aqueous solvent containing an electrolyte,followed by gelling, and a gel body includes a gel to a hard film.

In terms of the ion conductivity, safety, and the like, the acrylonitrile copolymer sheet soluble in a non-aqueous solvent is preferably composed of an acrylonitrile/C1 to C4 alkyl (meth)acrylate copolymer, an acrylonitrile/vinylacetate copolymer, an acrylonitrile/styrene copolymer, an acrylonitrile/vinylidene chloride copolymer, or the like. The copolymer sheet is made porous by an ordinary method such as a wet (dry) paper making method, a needlepunching method that is a kind of a non-woven fabric producing method, a water-j et method, drawing perforation of a melt-extruded sheet, or perforation by solvent extraction. In the present invention, among the above-mentioned ion conductive porous sheets of an acrylonitrile copolymer used in a solid-state battery, a gel body (a filmy body from a gel to a hard film) holding the above-mentioned aqueous solution in a three-dimensional network of a polymer chain and in which the above-mentioned impregnation ratio and transport number are achieved is useful as a thin film carrier used in the drug solution holding part 14 or the electrolyte solution holding parts 12, 22, and 24 of the present invention.

In the present invention, regarding the conditions for impregnating the above-mentioned thin film carrier with a drug solution or an electrolyte solution, the optimum conditions may be determined in terms of the impregnation amount, impregnation speed, and the like. For example, an impregnation condition of 30 minutes at 40°C may be selected.

An ion-exchange membrane carrying an ion-exchange resin having an anion exchange function in a base, for example, NEOSEPTA, AM-1, AM-3, AMX, AHA, ACH, ACS, ALE04-2, AIP-21, produced by Tokuyama Co., Ltd. can be used as each of the anion exchange membranes (ion-exchange membranes having characteristics of selectively passing minusions) 13 and 25 in the iontophoresis device X1 according to this embodiment. An ion-exchange membrane carrying an ion-exchange resin having a cation exchange function in a base, for example, NEOSEPTA, CM-1, CM-2, CMX, CMS, CMB, CLE04-2 produced by Tokuyama Co., Ltd. can be used as each of the cation exchange membranes (ion-exchange membranes having characteristics of selectively passing plus ions) 15 and 23. In particular, a cation exchange membrane in which a part or an entirety of pores of a porous film is filled with an ion-exchange resin having a cation exchange function, or an anion exchange membrane filled with an ion-exchange resin having an anion exchange function can be used preferably.

Herein, a fluorine type resin with an ion-exchange group introduced to a perfluorocarbon skeleton or a hydrocarbon type resin containing a resin that is not fluorinated as a skeleton can be used as the above-mentioned ion-exchange resin. In view of the convenience of a production process, a hydrocarbon type ion-exchange resin is preferable. Furthermore, although the filling ratio of the ion-exchange resin is also related to the porosity of the porous film, the filling ratio is generally 5 to 95% by mass, in particular, 10 to 90% by mass, and preferably 20 to 60% by mass.

There is no particular limit to an ion-exchange group of the above-mentioned ion-exchange resin, as long as it is a functional group generating a group having negative or positive charge in an aqueous solution. As specific examples of the functional group to be such an ion-exchange group, those of a cation exchange group include a sulfonic acid group, a carboxylic acid group, and a phosphonic acid group. Those acid groups may be present in the form of a free acid or a salt. Examples of a counter cation in the case of a salt include alkaline metal cations such as sodium ionsandpotassiumions,andammoniumions. Ofthosecationexchange groups, generally, a sulfonic acid group that is a strong acidic group is particularly preferable. Furthermore, examples of the anion exchange group include primary to tertiary amino groups, a quaternary ammonium group, a pyridyl group, an imidazole group, a quaternary pyridinium group, and a quaternary imidazolium group. Examples of a counter anion in those anion exchange groups include halogen ions such as chlorine ions and hydroxy ions. Of those anion exchange groups, generally, a quaternary ammonium group and a quaternary pyridinium group that are strong basic groups are used preferably.

A film shaped or a sheet shaped film having a number of small pores communicating a front surface and a back surface thereof are used as the above-mentioned porous film without any particular limit. In order to satisfy both the high strength and the flexibility, it is preferable that the porous film be made of a thermoplastic resin.

Examples of the thermoplastic resins constituting the porous film include, without limitation: polyolefin resins such as homopolymers or copolymers of α-olefins such as ethylene,propylene, 1-butene, 1-pentene, 1-hexene, 3-methyl-1-butene, 4-methyl-1-pentene, and 5-methyl-1-heptene; vinyl chloride resins such as polyvinyl chloride, vinyl chloride-vinyl acetate copolymers, vinyl chloride-vinylidene chloride copolymers, and vinyl chloride-olefin copolymers; fluorine resins such as polytetrafluoroethylene, polychlorotrifluoroethylene, polyvinylidene fluoride, tetrafluoroethylene-hexafluoropropylene copolymers, tetrafluoroethylene-perfluoroalkyl vinylether copolymers, and tetrafluoroethylene-ethylene copolymers; polyamide resins such as nylon 6 and nylon 66; and those which are made from polyamide resins. Polyolefin resins are preferably used as they are superior in mechanical strength, flexibility, chemical stability, and chemical resistance, and have good compatibility with ion-exchange resins. As the polyolefin resins, polyethylene and polypropylene are particularly preferable and polyethylene is most preferable.

There is no particular limit to the property of the above-mentioned porous film made of the thermoplastic resin. However, the average pore diameter of pores may be preferably 0. 005 to 5. 0 µm, more preferably 0.01 to 2.0 µm, and most preferably 0.02 to 0.2 µm since the porous film having such an average pore diameter is likely to beath in ion-exchange membrane having excellent strength and a low electric resistance. The average pore diameter in the present specification refers to an average flow pore diameter measured in accordance with a bubble point method (JISK3832-1990). Similarly, the porosity of the porous film may be preferably 20 to 95%, more preferably 30 to 90%, and most preferably 30 to 60%. Furthermore, the thickness of the porous film may be preferably 5 to 140 µm, more preferably 10 to 120 µm, and most preferably 15 to 55 µm. Usually, an anion exchange membrane or a cation exchange membrane using such a porous film has a thickness of the porous film with +0 to 20 µm.

As the porous separation membrane F1 of the iontophoresis device X1 according to this embodiment, a porous separation membrane made of an arbitrary material such as a porous film made of a polymer material such as polysulfone, polyacrylonitrile, cellulose acetate, polyamide, polycarbonate, or polyvinyl alcohol, or a porous film made of ceramics material (e.g., alumina) and having a number of pores communicating the front surface and the back surface of the film can be used. A porous separation membrane having appropriately sized pores can be selected to be used, depending upon the kind of an electrolyte dissolved in the electrolyte solution of the electrolyte solution holding part 12 or the kind of a drug dissolved in the drug solution of the drug solution holding part 14.

For example, in the case of using a sodium fumarate aqueous solution as the electrolyte solution of the electrolyte solution holding part 12, and using a lidocaine hydrochloride aqueous solution as the drug solution of the drug solution holding part 14, by leaving an assembled active electrode structure without the porous separation membrane F1 for a predetermined period of time (e.g., about several days), lidocaine ions that are drug ions are transferred to the electrolyte solution holding part 12, and sodium ions that are first electrolytic ions and fumaric acid ions that are the second electrolytic ions are transferred to the drug solution holding part 14. Depending upon the temperature condition, and the like, while the working electrode structure is left, the discoloration or alteration of the lidocaine hydrochloride aqueous solution or the precipitation of lidocaine hydrochloride crystal occurs. Furthermore, the administration efficiency of lidocaine ions at a time of administration of a drug decreases. Furthermore, depending upon the current passage condition at a time of the administration of a drug, lidocaine ions in the vicinity of the electrode 11 are decomposed.

However, by blocking the transfer of sodium fumarate molecules with a molecular weight of 137 and fumaric acid ions with a molecular weight of 115 to the drug solution holding part 14, and the transfer of lidocaine hydrochloride with a molecular weight of 268 (or lidocaine ions with a molecular weight of 234) to the electrolyte solution holding part 12, by using as the porous separation membrane F1 a porous separation membrane with a molecular weight cutoff of about 100 (for example, available as NUCLEPORE from Whatman plc or as Por™CE from Spectrum Laboratories, Inc.), the period during which the working electrode structure A1 can be retained is prolonged remarkably without the occurrence of the above-mentioned inconvenient phenomena such as the discoloration and alteration of a lidocaine hydrochloride aqueous solution, the precipitation of lidocaine hydrochloride crystal, or the decrease in an administration efficiency of lidocaine ions and the decomposition thereof. On the other hand, the transfer of chlorine ions that are drug counter ions to the electrolyte solution holding part 12 is not impaired. Therefore, the current passage characteristics required for the administration of a drug are ensured.

Even in the case of blocking the transfer of lidocaine hydrochloride molecules (or lidocaine ions) to the electrolyte solution holding part 12 by using, for example, a porous separation membrane with a molecular weight cutoff of about 200, as the porous separation membrane F1, the lidocaine ions in the vicinity of the electrode 11 are prevented from being decomposed in the case where a drug is administered after the retention of the working electrode structure for a long period of time. In addition, the transfer of fumaric acid ions and sodium fumarate molecules to the drug solution holding part 14 is blocked to some degree by the porous separation membrane F1. Therefore, it is possible to prolong the period during which the working electrode structure can be retained, without the occurrence of the phenomena such as the discoloration and alteration of a lidocaine hydrochloride aqueous solution, the precipitation of lidocaine hydrochloride crystal, or the decrease in an administration efficiency of lidocaine ions, to such a degree following the case of using the porous separation membrane F1 with a molecular weight cutoff of 100.

A battery, a voltage stabilizer, a current stabilizer (galvano device), a voltage/current stabilizer, or the like can be used as the power source C in the iontophoresis device of the present invention,. It is preferable to use a current stabilizer that is operated under safe voltage conditions in which an arbitrary current can be adjusted in a range of 0.01 to 1.0 mA, preferably 0.01 to 0.5 mA, specifically, at 50 V or less, preferably, 30 V or less.

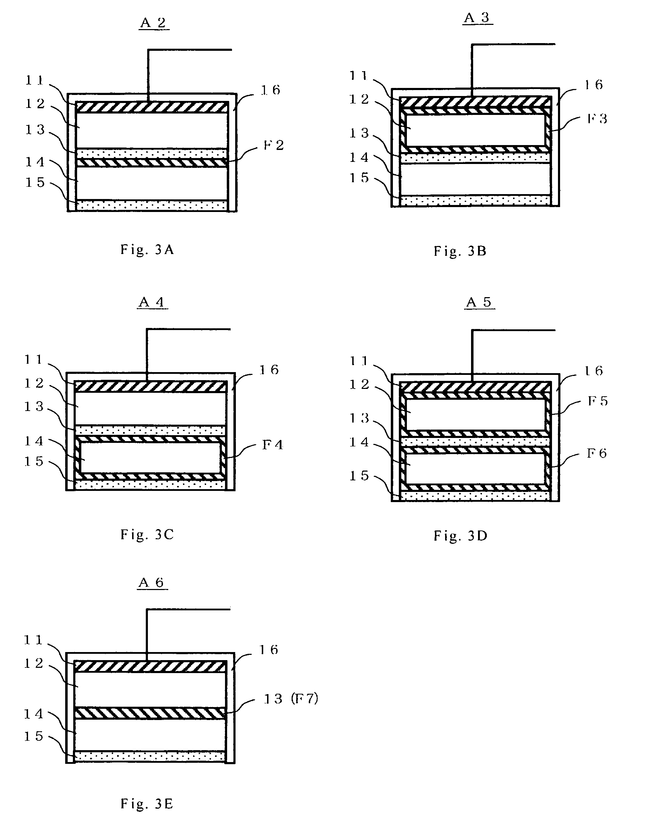

FIGS. 3A to 3D are explanatory views showing configurations of the working electrode structures A2 to A5 of the iontophoresis device according to another embodiment of the present invention.

As shown, the working electrode structure A2 has the same configuration as that of the working electrode structure A1 except that a porous separation membrane F2 is placed on a front side of the ion exchange membrane 13. An iontophoresis device using the working electrode structure A2 in place of the working electrode structure A1 achieves the same effect as that of the above-mentioned iontophoresis device X1.

The working electrode structure A3 has the same configuration as that of the working electrode structure A1 except that the electrolyte solution holding part 12 is sealed in a porous separation membrane F3 formed in a bag shape. The working electrode structure A4 has the same configuration as that of the working electrode structure A1 except that the drug solution holding part 14 is sealed in a porous separation membrane F4 formed in a bag shape. Furthermore, the working electrode structure A5 has the same configuration as that of the working electrode structure A1 except that the electrolyte solution holding part 12 is sealed in a porous separation membrane F5 formed in a bag shape, and the drug solution holding part 14 is sealed in the porous separation membrane F6 formed in a bag shape. Iontophoresis devices having the working electrode structures A3 to A5 achieve the same functional effect as that of the above-mentioned iontophoresis device X1, and also achieve the following additional functional effects: the mixing of an electrolyte solution and a drug solution is preventedmore exactly; and the handleability of the electrolyte solution holding part 12 and the drug solution holding part 14, and the workability for assembling the working electrode structures A3 to A5 are enhanced.

Even when the electrode 11 and/or the ion-exchange membrane 13 are/is further sealed in the porous separation membrane F3 of the working electrode structure A3, the ion-exchange membrane 13 and/or the ion-exchange membrane 15 are/is further sealed in the porous separation membrane F4 of the working electrode structure A4, or the electrode 11 and/or the ion-exchange membrane 13 are/is further sealed in the porous separation membrane F5 of the working electrode structure A5 and the ion-exchange membrane 13 and/or the ion-exchange membrane 15 are/is further sealed in the porous separation membrane F6 of the working electrode structure A5, the same functional effects as those described above are achieved.

FIG. 3E is an explanatory view showing the configuration of a working electrode structure A6 of the iontophoresis device according to still another embodiment of the present invention.

In the working electrode structure A6, an ion-exchange membrane 13 (F7) provided with the function of blocking the transfer of electrolyte molecules (or first electrolytic ions) and/or second electrolytic ions to the drug solution holding part 14, and/or the transfer of drug molecules (or drug ions) to the electrolyte solution holding part 12 is disposed in place of the ion-exchange membrane 13 and the porous separation membrane F1 of the working electrode structure A1. An iontophoresis device provided with the working electrode structure A6 achieves the same functional effect as that of the iontophoresis device X1.

An ion-exchange membrane similar to the ion-exchange membrane 13 used in the above-mentioned active electrode structure A1 can be used as the ion-exchange membrane 13 (F7). In the case of using an ion-exchange membrane in which a porous film having a number of pores communicating the front surface and the back surface is filled with ion-exchange resin, the function of blocking the transfer of the above-mentioned respective molecules or ions can be provided by adjusting the sizes of the pores and the ion-exchange resin, the filling amount of the ion-exchange resin, and the like.

The present invention has been described by way of some embodiments. The present invention is not limited to those embodiments, and variously altered within the scope of claims.

For example, in the above embodiments, the case where the working electrode structure has the second ion-exchange membrane 15 has been described as the most preferable embodiment. However, drug ions can also be administered under the condition that the second ion-exchange membrane 15 is omitted, and the drug solution holding part 14 is kept in direct contact with a living body.

Similarly, in the above embodiments, the case where the nonworking electrode structure B1 includes the electrode 21, the electrolyte solution holding parts 22 and 24, and the ion-exchange membranes 23 and 25 has been described. However, the ion-exchange membranes 23 and 25, and the electrolyte solution holding part 24 in the nonworking electrode structure B1 can be omitted. Alternatively, the following is also possible. The nonworking electrode structure is not provided in the iontophoresis device, and for example, under the condition that a part of a living body is kept in contact with a member to be the earth while the working electrode structure is kept in contact with the skin of the living body, a voltage is applied to the working electrode structure to administer a drug. Such an iontophoresis device is not comparable to the iontophoresis device X1 in terms of the performance of suppressing the change in pH on the contact surface of the skin S with respect to the nonworking electrode structure or the earth member. However, the iontophoresis device exhibits the same performance as that of the iontophoresis device X1 in the other points. In particular, the iontophoresis device exhibits the following effect peculiar to the present invention: thetransferofthe second electrolytic ions and/or the electrolyte molecules (or the first electrolytic ions) to the drug solution holding part and/or the transfer of drug molecules (or drug ions) to the electrolyte solution holding part are blocked, whereby the period during which the working electrode structure or the iontophoresis device can be retained is prolonged without the occurrence of the phenomena such as the discoloration, alteration, and decomposition of a drug, and the decrease in an administration efficiency of the drug. Those iontophoresis devices are also included in the scope of the present invention.

Furthermore, in each of the above embodiments, the case has been described where the working electrode structure, the non working electrode structure, and the power source are configured separately. It is also possible that those elements are incorporated in a single casing or an entire device incorporating them is formed in a sheet shape or a patch shape, whereby the handling thereof is enhanced, and such an iontophoresis device is also included in the scope of the present invention.

## Claims

1. An iontophoresis device (X1) for administering drug ions to a living body, comprising:
an electrolyte solution holding part (12) for holding an electrolyte solution in which an electrolyte to be dissociated, in a solution, to first electrolytic ions of a first conductivity and second electrolytic ions of a second conductivity opposite to the first conductivity is dissolved; and
a drug solution holding part (14) for holding a drug solution in which a drug to be dissociated, in a solution, to the drug ions of the first conductivity and drug counter ions of the second conductivity is dissolved, the drug solution holding part being placed on a front side of the electrolyte solution holding part (12),
**characterized in that** a first ion-exchange membrane (13) for selectively passing the ions of the second conductivity and a porous separation membrane (F1, F2, F3, F4, F5, F6, F7) for blocking passage of molecules and ions each having a predetermined molecular weight or more are placed between the electrolyte solution holding part (12) and the drug solution holding part (14).

2. An iontophoresis device (X1) according to claim 1, **characterized in that** the porous separation membrane (F1, F2, F3, F4, F5, F6, F7) blocks passage of the second electrolytic ions.

3. An iontophoresis device (X1) according to claim 1, **characterized in that** the porous separation membrane (F1, F2, F3, F4, F5, F6, F7) blocks passage of one of the electrolyte molecules and the first electrolytic ions.

4. An iontophoresis device (X1) according to claim 1, **characterized in that** the porous separation membrane (F1, F2, F3, F4, F5, F6, F7) blocks passage of one of the drug molecules and the drug ions.

5. An iontophoresis device (X1) according to any one of claims 1 to 4, **characterized in that** the electrolyte solution holding part (12) is sealed in the porous separation membrane (F3, F5) formed in a bag shape.

6. An iontophoresis device (X1) according to any one of claims 1 to 4, **characterized in that** the drug solution holding part (14) is sealed in the porous separation membrane (F2, F4) formed in a bag shape.

7. An iontophoresis device (X1) for administering drug ions to a living body, **characterized by** comprising:
an electrolyte solution holding part (12) for holding an electrolyte solution in which an electrolyte to be dissociated, in a solution, to first electrolytic ions of a first conductivity and second electrolytic ions of a second conductivity opposite to the first conductivity is dissolved;
a drug solution holding part (14) for holding a drug solution in which a drug to be dissociated, in a solution, to the drug ions of the first conductivity and drug counter ions of the second conductivity is dissolved, the drug solution holding part (14) being placed on a front side of the electrolyte solution holding part (12); and
a first ion-exchange membrane (13(F7)) for selectively passing ions of the second conductivity, the first ion-exchange membrane (13(F7)) being placed between the electrolyte solution holding part (12) and the drug solution holding part (14),
wherein the first ion-exchange membrane (13(F7)) blocks passage of the second electrolytic ions.

8. An iontophoresis device (X1) for administering drug ions to a living body, **characterized by** comprising:
an electrolyte solution holding part (12) for holding an electrolyte solution in which an electrolyte to be dissociated, in a solution, to first electrolytic ions of a first conductivity and second electrolytic ions of a second conductivity opposite to the first conductivity is dissolved;
a drug solution holding part (14) for holding a drug solution in which a drug to be dissociated, in a solution, to the drug ions of the first conductivity and drug counter ions of the second conductivity is dissolved, the drug solution holding part (14) being placed on a front side of the electrolyte solution holding part (12); and
a first ion-exchange membrane (13(F7)) for selectively passing ions of the second conductivity, the first ion-exchange membrane being placed between the electrolyte solution holding part (12) and the drug solution holding part (14),
wherein the first ion-exchange membrane (13(F7)) blocks passage of one of the electrolyte molecules and the first electrolytic ions.

9. An iontophoresis device (X1) for administering drug ions to a living body, **characterized by** comprising:
an electrolyte solution holding part (12) for holding an electrolyte solution in which an electrolyte to be dissociated, in a solution, to first electrolytic ions of a first conductivity and second electrolytic ions of a second conductivity opposite to the first conductivity is dissolved;
a drug solution holding part (14) for holding a drug solution in which a drug to be dissociated, in a solution, to the drug ions of the first conductivity and drug counter ions of the second conductivity is dissolved, the drug solution holding part (14) being placed on a front side of the electrolyte solution holding part (12); and
a first ion-exchange membrane (13(F7)) for selectively passing ions of the second conductivity, the first ion-exchange membrane (13(F7)) being placed between the electrolyte solution holding part (12) and the drug solution holding part (14),
wherein the first ion-exchange membrane (13(F7)) blocks passage of one of the drug molecules and the drug ions.

10. An iontophoresis device (X1) according to any one of claims 1 to 9, **characterized by** further comprising a second ion-exchange membrane (15) for selectively passing the ions of the first conductivity, the second ion-exchange membrane (15) being placed on a front side of the drug solution holding part (14).
